# EUROPEAN PATENT APPLICATION

(11) **EP 3 699 297 A1**
(43) Date of publication of application: **26.08.2020**
(21) Application number: 19159068.6
(22) Date of filing: 25.02.2019
(51) Int. Cl.: C12Q 1/6834, C12Q 1/6823, C12Q 1/6853

(54) **A METHOD FOR DETERMINING THE LEVEL OF AN ANTISENSE OLIGONUCLEOTIDE**

(71) Applicant: QIAGEN GmbH, 40724 Hilden (DE)
(72) Inventor: The designation of the inventor has not yet been filed
(74) Representative: Banse & Steglich Patentanwälte PartmbB

(57) **Abstract**

Subject of the invention is a method for determining the level of an antisense oligonucleotide in a biological sample, comprising the steps of
(A) providing a sample, which is the biological sample or a fraction thereof,
(B) adding a first nucleic acid to the sample, which is capable of hybridizing with the antisense oligonucleotide,
(C) forming a hybridization product of the antisense oligonucleotide and the first nucleic acid,
(D) selectively separating the hybridization product from the biological sample,
(E) performing a polymerase chain reaction (PCR) to obtain an amplified nucleic acid, and
(F) determining, based on the level of the amplified nucleic acid, the level of the antisense oligonucleotide in the biological sample,
wherein the hybridization product in step (C) does not comprise two contiguous sequences of nucleotides bound to the antisense oligonucleotide, which are interrupted by a nick. The invention also relates to a kit for carrying out the method.

## Description

The invention relates to methods and kits for determining the level of antisense oligonucleotides in a biological sample.

### State of the art

Antisense oligonucleotides are synthetic single-stranded nucleic acid molecules that are capable of binding to complementary target RNA molecules by hybridization. Thereby, antisense oligonucleotides interfere with gene expression. Antisense oligonucleotides play an increasing role as drugs in antisense therapy. They have been developed for treatment of diseases such as various cancers, diabetes, amyotrophic lateral sclerosis (ALS), Duchenne muscular dystrophy, spinal muscular atrophy, asthma and arthritis. Several antisense drugs have been approved by the US Food and Drug Administration.

Due to their unique chemical structure, antisense oligonucleotides have fundamentally different physical and chemical properties than typical small molecule drugs. Due to the negative charge, it is difficult to mediate uptake through cell membranes and biodistribution can be unfavorable. Therefore, in drug development, but also general research, it is important to determine levels of antisense oligonucleotides in biological materials, such as tissue, body fluids or cells.

In the prior art, antisense oligonucleotides in biological samples are normally detected by fluorescence-based methods, wherein fluorescent labels are attached to the oligonucleotides. Only as an example, Hvam et al., Molecular Therapy, Vol. 25(7), 2017, 1710-1717, discloses methods for determining antisense oligonucleotide levels by fluorescence. However, fluorescence-based detection methods have various limitations and drawbacks. The use of fluorescent labels is not desirable for *in vivo* experiments, especially clinical trials, for safety reasons. Further, the sensitivity of fluorescence-based assays can be insufficient, especially when only small amounts of antisense oligonucleotides are present in a sample. Fluorescence-based methods can also be imprecise, because oligonucleotides can be partly degraded or modified *in vivo.* In such cases, false positives may result from non-functional fluorescent oligonucleotide fragments. Moreover, synthesis of fluorescence-labelled oligonucleotides can be relatively complicated.

In order to overcome the drawbacks of the fluorescent detection methods, other methods have been suggested, which do not use fluorescent markers. In this regard, WO2019/011875 A1 discloses a method in which a sample comprising an oligonucleotide is provided and the oligonucleotide is hybridized, such that the complementary strain in the hybridization product is interrupted by a nick. The nick is closed in a subsequent ligation reaction and the amount of oligonucleotide in the sample can be determined based on an amplification reaction.

Overall, there is a continuous need for new and improved methods for the quantification of antisense oligonucleotides in biological samples, which overcome the above mentioned drawbacks.

### Problem underlying the invention

It is an aim underlying the invention to provide new methods for determining the level of antisense oligonucleotides in biological samples which overcome the above mentioned drawbacks. It shall be applicable with a relatively low number of process steps and with a low number of reagents and tools, such as nucleic acids and enzymes.

It is a specific aim to provide an efficient method for determining the level of gapmers. The method shall preferably be applicable for high throughput methods with a large number of samples and automated devices, such as robotic work stations. It is a specific aim to provide a method which does not require relatively complicated intermediates.

### Disclosure of the invention

Surprisingly, it was found that the problem underlying the invention is overcome by methods and kits according to the claims. Further embodiments of the invention are outlined throughout the description.

### General description of the method

Subject of the invention is a method for determining the level of an antisense oligonucleotide in a biological sample, comprising the steps of
(A) providing a sample, which is the biological sample or a fraction thereof,
(B) adding a first nucleic acid to the sample, which is capable of hybridizing with the antisense oligonucleotide,
(C) forming a hybridization product of the antisense oligonucleotide and the first nucleic acid,
(D) selectively separating the hybridization product from the biological sample,
(E) performing a polymerase chain reaction (PCR) to obtain an amplified nucleic acid, and
(F) determining, based on the level of the amplified nucleic acid, the level of the antisense oligonucleotide in the biological sample,
wherein the hybridization product in step (C) does not comprise two contiguous sequences of nucleotides bound to the antisense oligonucleotide, which are interrupted by a nick.

As used herein, "determining the level" means quantification. Since the level is determined based on PCR, a relatively precise quantification is enabled. However, the level can also be determined superficially, for example visually.

The method is carried out with the biological sample or a fraction thereof. In a preferred embodiment, the biological sample is selected from tissue, body fluids, such as blood, saliva, urine or liquor, and cells. For example, the sample can be a tissue biopsy, blood, a blood fraction, such as serum or plasma, or tissue from animals or plants. The biological sample may be pre-treated, for example by lysis of cellular components according to standard methods, or by mixing with additives, such as proteases, RNases, stabilizers or inhibitors.

An "antisense oligonucleotide" is a single-stranded oligonucleotide, which is capable of hybridization to a nucleic acid from the organism, from which the biological sample is obtained. The antisense oligonucleotide may have a length of 10 to 50 nucleotides, preferably 12 to 30, or more preferably 15 to 25 nucleotides. The antisense oligonucleotide may be a DNA and/or RNA, optionally comprising at least one modified nucleosidic building block and/or at least one modified internucleosidic linkage between two nucleoside building blocks. It is preferred that the antisense oligonucleotide is not entirely a RNA. However, it may comprise one or more RNA segments.

Preferably, the antisense oligonucleotide is an oligodeoxyribonucleotide comprising at least one modified nucleoside building block and/or at least one modified internucleosidic linkage. The presence of modified nucleoside building blocks and/or modified internucleosidic linkages increases the stability of antisense oligonucleotides under physiological conditions. Thus, pharmaceutical antisense oligonucleotides usually comprise such modifications. Examples of modified nucleoside building blocks are base-modified building blocks comprising a non-naturally occurring nucleobase, e.g. 5-methyl cytosine, and sugar-modified building blocks comprising a non-naturally occurring sugar moiety, e.g. a 2'-modified sugar moiety and/or a locked sugar moiety. Examples of 2'-modified nucleoside building blocks are 2'-methoxy, 2'-F and/or 2'-O-ethoxymethyl building blocks. Locked nucleoside building blocks contain a bridge between two atoms of the pentose sugar, e.g. the deoxyribose sugar. For example, a bridge is formed between the 2'-C atom and the 4'-C atom of the sugar, e.g. a 2-atom or 3-atom bridge, preferably comprising at least one heteroatom such as O, N or S. Preferred are bridges having the structure 2'-O-CH₂-4' or 2'-O-CH₂-CH₂-4', wherein one or more H atoms may be substituted, e.g. by C₁-C₃ alkyl, C₁-C₃ alkoxy groups, e.g. a 2'-O-CH(CH₃)-4' bridge. Especially preferred is a bridge having the structure 2'-O-CH₂-4'. The internucleosidic linkages in the oligonucleotide analyte may be phosphodiester bonds and/or modified internucleosidic linkages such as phoshorothioate linkages. In many cases, antisense oligonucleotides comprise a plurality of modified nucleoside building blocks, e.g. locked nucleoside building blocks and/or 2'- modified building blocks and a plurality of modified internucleosidic linkages. Modified nucleoside building blocks and/or modified nucleosidic linkages may be present at any position of the antisense oligonucleotide. The respective molecules are designated as gapmer, blockmer, headmer, tailmer, mixmer or totalmer. An overview of antisense oligonucleotides, uses and methods for their production is provided by Shen and Corey, Nucleic Acids Research, 2018, 46(4), 1584-1600.

In the method, the level of a specific antisense oligonucleotide of known structure is determined. The method is specific for this antisense oligonucleotide and does not at the same time determine the level of other oligonucleotides, such as mRNAs.

In a preferred embodiment, the first nucleic acid comprises a label for selective binding. A label is a moiety covalently attached to the nucleic acid, optionally through a linker. Selective means that only the labelled molecule is bound, but not other unlabelled molecules.

Preferably, the label is for affinity binding, such as a biotin label, or for covalent binding. The label is preferably a tag, such as a peptide or polynucleotide tag, which is capable of specific binding to a known binding partner with high affinity. Such labels and tags are known in the art and can be covalently attached to the nucleic acid by known methods. In a preferred embodiment, the label is a protein tag, such as biotin, which is capable of high affinity binding to streptavidin or a streptavidin-labelled support. Kits and tools for labelling nucleic acid molecules with affinity labels and tags, such as biotin and streptavidin labels, are available in the art, for example from ThermoFisher, U.S.

In step (B), the first nucleic acid is added to the sample. The first nucleic acid is capable of hybridizing with the antisense oligonucleotide. In step (C), conditions are adjusted such that a hybridization product of the antisense oligonucleotide and the first nucleic acid is formed. Preferably, the first nucleic acid is selected such that the hybridization product is sufficiently stable at room temperature (22°C). Stable hybridization is generally achieved, when the antisense oligonucleotide and in the first nucleic acid comprise a complementary nucleic acid sequences of sufficient length. Preferably, the length of the complementary sequence is at least 8 or at least 10 nucleotides, preferably between 8 and 30, more preferably between 10 and 25 nucleotides.

The first nucleic acid in step (B) can be a single-stranded nucleic acid or a nucleic acid strand of a duplex nucleic acid, especially duplex DNA. When the first nucleic acid is part of a duplex nucleic acid, the temperature is temporarily increased after addition, such that the duplex is denatured and the single stranded first nucleic acid is set free.

In step (C), conditions are adjusted for hybridization, typically by temperature reduction. Overall, the temperature is controlled such that the hybridization product in step (C) can be formed. Preferably, formation of the hybridization product is quantitative, such that essentially all antisense oligonucleotide, or at least more than 90%, more than 94% or more than 98%, become part of a hybridization product.

Antisense oligonucleotide hybridization can be supported by adding a large excess of the first nucleic acid, for example at least 10fold or 100fold molar excess, and/or when the first nucleic acid has high affinity to the antisense oligonucleotide, for example due to a complementary region of at least 10 or at least 15 nucleotides.

Step (D) of separating the hybridization product is carried out under conditions in which the hybridization product is sufficiently stable, especially temperature, but also pH and the like.

In a preferred embodiment, in step (D) the hybridization product is separated from the sample by selective binding, especially by affinity binding or covalent binding. The hybridization product can be separated conveniently when the first nucleic acid, which is part of the hybridization product, comprises a label for affinity binding or covalent binding. Thereby, the hybridization product, which includes the antisense oligonucleotide, can be separated from the biological sample. In a preferred embodiment, the hybridization product is bound to a solid support, for example to beads, such as magnetic beads. Preferably, the antisense oligonucleotide comprises a biotin label, and the hybridization product is separated by affinity binding to streptavidin beads, preferably magnetic beads. Kits and tools for labelling nucleic acid with streptavidin and biotin are commercially available, for example from Qiagen, DE, under the trademark BioMag Strepatividin.

Removal of the hybridization product from the biological sample is advantageous, because the subsequent PCR reaction must not be carried out in the biological sample. Since a biological sample comprises normally a large number of molecules, including RNA and DNA, such a method can be more precisely compared to a method in which PCR is carried out in the biological sample. False positive or negative results due to side reactions or binding of antisense oligonucleotide to other nucleic acids in the sample can be avoided.

Although an affinity binding system, such as the biotin/streptavidin system, preferably in combination with magnetic beads, is preferred due to its high efficacy and binding strength, another known binding system for biological molecules in the art can also be used. In another embodiment, a covalent binding system could be used. In such a reaction, a covalent bond is formed, which can be cleaved under defined conditions. It is advantageous that the covalent binding strength is especially high, such that a loss of target molecules is kept low. Preferably, the covalent binding system is for immobilization by "click chemistry". For example, a 5' terminal azid and/or alkyne modification could be used.

In step (E), a PCR reaction is carried out. In step (F), the level of antisense nucleotide is determined based on the result of the PCR, i.e. the level of the amplified nucleic acid. Thus, the target DNA of the PCR is a polynucleotide which is indicative of the amount of antisense oligonucleotide in the biological sample. In principle, it may be considered to amplify the antisense oligonucleotide directly by PCR. However, this may be difficult because of the small size of the antisense oligonucleotide. Preferably, the target DNA of the PCR is not the antisense oligonucleotide itself, but a different nucleic acid molecule, which provides information about the level of antisense oligonucleotide in the biological sample.

In a preferred embodiment, the amount of the target DNA for the PCR is equivalent to the amount of antisense oligonucleotide in the sample. This can be achieved by specific methods described further below, in which exactly one nucleic acid molecule, preferably a DNA, is synthesized per antisense oligonucleotide, which is more stable and/or longer, such that it can be analyzed more conveniently. Preferably, the amount of the target DNA of the PCR corresponds to the amount of antisense oligonucleotide in the hybridization product, i.e. is the same amount. Preferably, the PCR target DNA is at least in part homologous or complementary to the antisense oligonucleotide. For example, such a DNA can provide information about the level of the antisense oligonucleotide in the biological sample, if the method comprises a step of synthesis of the target DNA with the antisense oligonucleotide or a derivative thereof as a template, or competitive binding of the target DNA and the antisense oligonucleotide to another nucleic acid.

The PCR in step (E) can be carried out by known methods. Primers are constructed and added, which specifically amplify the target sequence. Preferably, the PCR is quantitative and/or real-time PCR, more preferably quantitative real-time PCR. This method is suitable for precise and rapid quantification of nucleic acids. Based on the result of the PCR and the amount of amplified nucleic acid, the level of antisense oligonucleotide in the biological sample is calculated. Preferably, the result is compared to a standard. For example, the method can be carried out in parallel with samples comprising known amounts of the antisense oligonucleotide to provide standard comparative results.

Preferably, one or more washing steps are carried out after each step, especially after step (D) and after removal of other components from the sample.

The inventive method is different from the method of WO2019/011875 A1, because the hybridization product in step (C) does not comprise two contiguous sequences of nucleotides bound to the antisense oligonucleotide, which are interrupted by a nick. Thus, it is not necessary in the inventive method to carry out a ligation reaction. According to the invention, the hybridization product can be formed from 2 nucleic acids and complementary sequences, and it is not necessary that 3 different nucleic acids and/or complementary sequences participate.

In a preferred embodiment, the method is carried out as an automated process, preferably by a robotic work station. Preferably, the method is carried out with a large number of samples in parallel, for example between 20 and 5000 samples, preferably 50 to 1000 samples, such as 96 samples. It is advantageous that the inventive method can be carried out as an automated process and with a high throughput, because it is based on standard enzymes, chemicals and conventional method steps, which can be automated without problems.

In the following, two preferred embodiments of the inventive method are described in more detail.

### Quantification of displaced DNA strand

Subject of the invention is also a method for determining the level of an antisense oligonucleotide in a biological sample, comprising the steps of
(A) providing a sample, which is the biological sample or a fraction thereof,
(B) adding a duplex DNA to the sample, the duplex DNA comprising a first and second DNA strand, wherein the first strand is capable of hybridizing with the antisense oligonucleotide, wherein the duplex DNA is added in a molar excess over the antisense oligonucleotide,
(C) forming a hybridization product of the antisense oligonucleotide with the first DNA strand, such that also a displaced second DNA strand is formed,
(D) selectively separating the hybridization product from the sample,
(E) performing a polymerase chain reaction (PCR), wherein the target is the second strand of the duplex DNA, and
(F) determining, based on the result of the PCR, the level of the antisense oligonucleotide in the biological sample.

This embodiment of the method is based on a competitive binding of the antisense oligonucleotide to a first strand of a duplex DNA, by which a displaced single strand of the duplex DNA is released into the sample. Thus, the molar amount of hybridized antisense oligonucleotide is the same as the molar amount of displaced second DNA strand. When the antisense oligonucleotide is hybridized quantitatively in the hybridization product, the amount of a displaced second DNA strand is indicative of the original amount of free antisense oligonucleotide in the sample.

Generally, quantitative formation of the hybridization product, which includes the antisense oligonucleotide, is highly desirable in this embodiment. Antisense oligonucleotide hybridization can be supported by adding a large excess of the duplex DNA and/or when the first strand of the duplex DNA has higher affinity to the antisense oligonucleotide than to the second strand of the duplex. When adjusting the method accordingly, the amount of displaced second DNA strand can provide indirect, precise and reliable information about the total amount of antisense oligonucleotide in the original biological sample.

Preferably, the antisense oligonucleotide has a relatively high affinity to DNA. Preferably, the antisense oligonucleotide comprises desoxynucleotides. Preferably, they comprise modified nucleotides, which enhance the affinity to DNA, such as LNA (locked nucleic acids), characterized by a 2',4'-methylene linkage, or (S)-cET (2',4'-constrained ethyl nucleic acid). In a highly preferred embodiment, the antisense oligonucleotide is a gapmer, which is a hybrid of two RNA segments flanking a central DNA segment. Such antisense oligonucleotides have especially high affinity to DNA and would lead to efficient displacement of the second DNA strand from the duplex DNA.

In a preferred embodiment, the first strand of the duplex DNA comprises chemically modified nucleotides with high binding affinity to the antisense oligonucleotide. Preferably, the antisense oligonucleotide comprises one or more, preferably from 2 to 10, more preferably from 4 to 8 locked nucleic acids (LNA). Preferably, the first strand of the duplex DNA also comprises LNA at corresponding positions. In this regard, corresponding positions mean complementary positions, such that the LNA of both strands form base pairs in the hybridization product. Preferably, the antisense oligonucleotide and/or the first strand of the duplex DNA, most preferably both, comprise 2 to 10, preferably between 4 to 8 LNAs at corresponding positions. Such LNAs increase the melting temperature (Tₘ) of the hybridization product of the antisense oligonucleotide with the first DNA strand. Preferably, the Tₘ of the hybridization product is significantly higher compared to a respective hybridization product without such LNA modifications, for example by more than 10°C, more than 20°C or even more than 30°C. In these embodiments with LNA, the displaced second DNA strand does not comprise LNA. Accordingly, the LNA in the antisense oligonucleotide and first DNA strand support quantitative formation of the hybridization product and displacement of the second DNA strand. More preferably, the duplex DNA comprises a first strand which is a biotin-labeled LNA/DNA mixmer complementary to the antisense oligonucleotide.

In a preferred embodiment, in step (B) the first strand of the duplex DNA comprises a first label for affinity binding, preferably a biotin label. In a preferred embodiment, in step (D) the hybridization product and duplex DNA are separated from the sample by a first affinity binding step. As outlined above for the general method, the hybridization product can be separated conveniently from the sample in step (D) with such an affinity binding label. In this embodiment, in step (D) the hybridization product and the duplex DNA are separated from the sample, because they both comprise the affinity label. Only the displaced second DNA strand is not bound in such a binding step and remains in the sample.

In step (E), a PCR is performed, wherein the target is the second strand of the duplex DNA. In step (F), the amount of antisense oligonucleotide in the biological sample is determined based on the result of the PCR. This is convenient, because the amount of displaced second strand DNA corresponds to the amount of antisense oligonucleotide in the biological sample.

In principle, the PCR in step (E) can be carried out with the remaining biological sample, from which the hybridization complex has been separated, and in which the displaced second strand of duplex DNA is comprised. In order to enhance the accuracy, it is preferred to selectively separate the displaced second DNA strand from the sample before the PCR reaction. Preferably, the second DNA strand is separated by affinity binding.

Preferably, after removal of the displaced second strand of duplex DNA before step (E), the second strand is eluted and PCR reagents are added directly to the eluate.

In a preferred embodiment, the second strand of the duplex DNA comprises a second label for affinity binding, preferably a poly-dA-tail. In a preferred embodiment, in a step (E0) before step (E), the displaced second strand of the duplex DNA is separated from the sample by a second affinity binding step by binding of the poly (A) tail to a poly(T)-affinity support. Preferably, the poly(A) tail labelled second DNA strand is isolated by binding to poly-dT bound to a solid support, preferably magnetic beads. Methods and kits for affinity binding of nucleic acids comprising poly(A) tails, are available in the art, for example from Qiagen, DE. Preferably, the sample is initially treated with an enzyme to separate linear RNA, such as RNase A. Typically, antisense oligonucleotides are designed such that they have a high binding affinity to RNA. Therefore, the RNase A treatment is advantageous for preventing antisense oligonucleotides binding to RNA rather than to the duplex DNA. The RNase A treatment should also digest RNA in complexes with antisense oligonucleotides, such that the antisense oligonucleotides are liberated for their quantification. In these embodiments, the RNase A does not cleave the antisense oligonucleotide. Further, proteinase K inhibitor may be added to the sample to protect RNase A from degradation. In another embodiment, a large surplus of RNase A is added, and no proteinase K inhibitor is added.

### Cleavage of hybridization product bv endonuclease

Subject of the invention is also a method for determining the level of an antisense oligonucleotide in a biological sample, wherein the antisense oligonucleotide comprises a DNA segment, comprising the steps of
(A) providing a sample, which is the biological sample or a fraction thereof,
(B) adding a single stranded RNA to the sample,
(C) forming a hybridization product from the antisense oligonucleotide and the single stranded RNA,
(D) selectively separating the hybridization product from the sample,

   (D1) contacting the hybridization product with an endonuclease, which specifically cleaves the single stranded RNA in the hybridisation product, but not the single stranded RNA which is not part of a hybridization product,
(E) performing a polymerase chain reaction (PCR), and
(F) determining, based on the result of the PCR, the level of the antisense oligonucleotide in the biological sample.

In this embodiment of the inventive method, a hybridization product of the antisense oligonucleotide with a synthetic single-stranded RNA is formed and isolated. Subsequently, the isolated hybridization product is treated with an endonuclease, such that only the single stranded RNA strand in the hybridization product, but not the antisense oligonucleotide is cleaved. Further, the single stranded RNA, which is not part of the hybridization product, is not cleaved by the endonuclease. This can be achieved with a hybridization product comprising a complementary RNA and DNA segment and an endonuclease which specifically cleaves RNA strands of RNA/DNA hybridization products. Thereby, an isolated truncated RNA is obtained, the level of which corresponds to the level of antisense oligonucleotide. The amount of truncated RNA can be determined by PCR after conversion into a successor DNA which can serve as a PCR substrate. For example, this can be achieved by cDNA synthesis with the truncated RNA as a template.

In principle, any antisense oligonucleotide can be analyzed by this embodiment of the method, which comprises a DNA segment of sufficient length, such as at least 7, 8 or 10 nucleotides. The DNA segment may comprise oligonucleotides with chemical modifications.

In a preferred embodiment, the endonuclease is RNase H (Ribonuclease H). RNase H is selective for cleaving RNA which is hybridized to DNA, whilst leaving the DNA unaffected. In therapy, RNase H induced cleavage, mediates destruction of target mRNA and is a key feature of the therapeutic effect and translation inhibition. Antisense oligonucleotides comprising DNA segments and gapmers are known in the art (Shen and Corey, 2018). The enzyme is known in the art and commercially available, for example from New England Biolabs, U.S.

In a preferred embodiment, the antisense oligonucleotide is a gapmer. Gapmers contain chemically modified RNA bases flanking both sides of a central DNA segment, which often comprises 7 to 12, preferably 8 to 10 bases. The RNA bases may contain modifications that enhance affinity to complementary sequences. The central DNA segment binds to RNA and provides a target site for endonuclease RNase H mediated cleavage.

In a preferred embodiment, the single stranded RNA comprises a label for affinity binding, preferably a biotin label. In a preferred embodiment, in step (D) the hybridization product is separated from the sample by affinity binding. Labels and methods for affinity binding can be selected and applied as outlined further above. In this embodiment of the method, the affinity label is not only attached to the hybridization product, but also to free single-stranded RNA which does not form a hybridization product with an antisense oligonucleotide. Therefore, when the hybridization product is separated from the sample by affinity binding in step (D), the free single-stranded RNA is also removed and bound to the affinity support.

In cleaving step (D1), the conditions, such as time and amount of endonuclease, preferably RNase H, are preferably controlled such that each captured hybridization product is cleaved only once on average. This is advantageous, because the chances are then lower that the antisense oligonucleotide, which should not be cleaved and may be released, binds to another single-stranded RNA, which is cleaved again. Such a side reaction would lead to an undesired higher amount of cleaved single stranded RNA.

Preferably, step (D1) is carried out when the isolates from step (D) are still bound to the affinity support. Thus, after step (D1) the cleaved RNA intermediate and the excess of original single-stranded RNA can still be bound to the support. At that stage, after optionally washing steps, an elution can be carried out such that an eluate comprising the labelled RNA molecules is obtained.

In a preferred embodiment, the single stranded RNA comprises a modification at the 3'-end which prevents polyadenylation. For example, the 3'-end modification is a dideoxycytidine. Such modifications and methods for producing modified nucleotides and nucleic acids are known in the art. Preferably, after step (D1) the cleaved single stranded RNA is then polyadenylated at the 3'-end. As known in the art, single-stranded RNA can be polyadenylated by polyadenylate polymerase in the presence of an appropriate reaction mixture. However, only the cleaved single-stranded RNA intermediate is polyadenylated. In contrast, the original single-stranded RNA, which was not cleaved, is not polyadenylated, because this is inhibited by the 3' end modification. Thus, the amount of polyadenylated RNA corresponds to the amount of antisense oligonucleotide in the sample.

As noted above, the amount of cleaved RNA intermediate obtained in step (D1), and thus also the amount of polyadenylated RNA, correlate to the level of antisense oligonucleotide in the original sample. Subsequently, it is preferred to convert the polyadenylated RNA into a suitable PCR template. In a preferred embodiment, cDNA is synthesized from the polyadenylated cleaved single stranded RNA and the cDNA is used as the template in the PCR reaction in step (E). Accordingly, cDNA is obtained only from cleaved single-stranded RNA which is derived from hybridization products, but not from the original free RNA which did not form hybridization products. Therefore, the amount of cDNA correlates to the amount of antisense oligonucleotide in the original sample. cDNA synthesis from RNA targets with poly(A) tails can be carried out by conventional methods in the presence of reverse transcriptase and a poly-dT RT primer with a universal sequence tag. Typically, at the 5'-end of the poly-dT stretch of the cDNA primer, there is a universal sequence which defines the reverse PCR primer. A second PCR primer is added, which corresponds to the 3-end of the cDNA. Enzymes, kits and tools for cDNA synthesis with universal primers for subsequent PCR analysis are commercially available, for example from Roche, CH.

Subject of the invention is also a kit for determining the level of an antisense oligonucleotide in a biological sample by an inventive method as described above, comprising
(i) a first nucleic acid, which is capable of hybridizing with the antisense oligonucleotide and/or means for its synthesis or modification, and
(ii) enzymes, reagents and/or devices for carrying out the method.

The kit comprises an instruction manual for carrying out the method. The enzymes, reagents and devices could be any of those described above, such as RNAse H. The first nucleic acid may be designed and/or may comprise modifications as outlined above.

The inventive method solves the problem defined above. A convenient and reliable method is provided by which the level of antisense oligonucleotides in a sample can be determined. The method is PCR-based and thus highly sensitive. In the method, the PCR target molecule is not part of the original sample. In contrast, the PCR target, or even a precursor thereof, is separated from the sample at an early phase of the process. Thereby, it is possible to determine the amount of antisense oligonucleotide in a precise and highly sensitive manner. Further, the overall process can be carried out with standard enzymes and equipment and with a relatively low number of process steps. The method is especially applicable for quantifying small antisense oligonucleotides such as gapmers, for which routine quantification is normally very difficult. It is another advantage that the method can be carried out in an automated process with a high number of probes.

Exemplified embodiments of the invention and aspects of the invention are shown in the figures.
Figure 1 shows schematically and in exemplified form an embodiment of the inventive method with quantification of a displaced DNA strand.
Figure 2 shows schematically and in exemplified form an embodiment of the invention with cleavage of a hybridization product by endonuclease.

### Examples

The following examples describe embodiments of the invention in exemplified form. The single steps can be adjusted and modified as outlined above.

### Example 1:

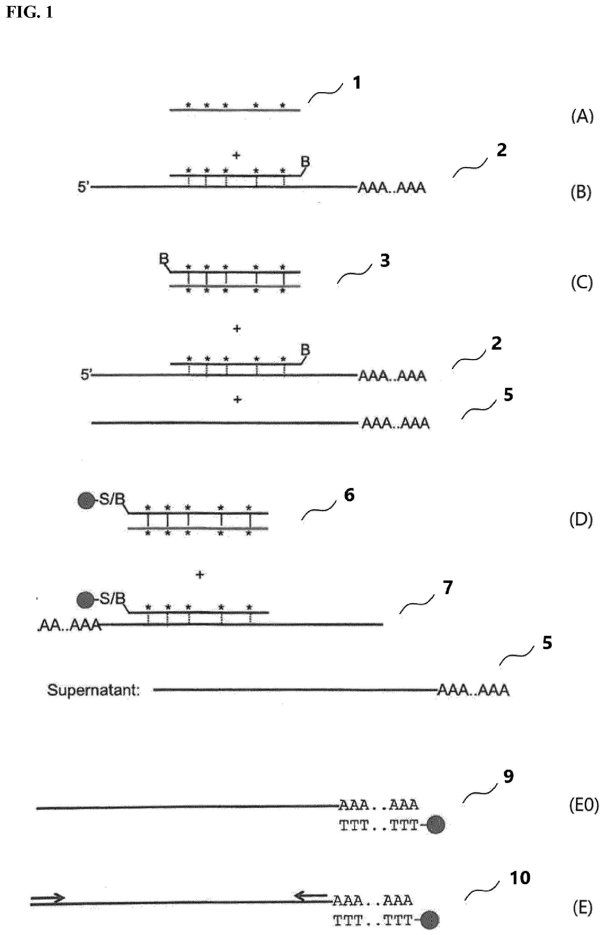

Fig. 1 shows a reaction scheme for an embodiment of the inventive method with quantification of a displaced DNA strand. In step (A), a cell lysate containing antisense oligonucleotide 1 is provided, which was treated with PMSF (proteinase K inhibitor) and RNase. The antisense oligonucleotide is a gapmer comprising a central DNA segment which comprises 5 LNA nucleotides (asterisks). In step (B), DNA duplex 2 is added, which consists of a first strand comprising a biotin label ("B"). The duplex DNA is added in an excess amount. The first strand is capable of hybridizing to the antisense oligonucleotide. It comprises a complementary sequence and LNA nucleotides at corresponding positions in order to achieve a high binding strength. The second strand of the DNA duplex comprises a poly(dA) tail of about 25 consecutive dA at the 3'-end. In step (C), a hybridization product 3 of the antisense oligonucleotide and the first DNA strand is formed by denaturing due to heating and annealing due to temperature reduction. The sample also comprises excess duplex DNA 2 and displaced single second strand DNA 5. In step (D), hybridization product bound to magnetic streptavidin beads 6 is selectively removed from the sample. However, duplex DNA in the sample is also biotin-tagged and also binds to streptavidin beads 7. Only displaced single-stranded second DNA 5 remains unbound in the sample. The magnetic beads are removed from the sample with bound hybridization product 6 and bound duplex DNA 7 and discarded. In step (E0), single-stranded second DNA 9 is selectively removed from the remaining sample by binding of its poly(dA) tail to poly(dT) beads, followed by washing. In step (E), quantitative PCR is performed with the single-stranded second DNA target bound to poly(dT)-beads in the presence of appropriate primer. Alternatively, the single-stranded second DNA can be eluted from the poly(dT)-beads and the PCR can be carried out in a separate device. The amount of antisense oligonucleotide in the original biological sample is determined based on the amount of displaced single-stranded second DNA, the amount of which corresponds to the original antisense oligonucleotide amount. For precise quantification, parallel reactions can be performed with a series of comparative probes comprising different pre-adjusted levels of the antisense oligonucleotide.

### Example 2:

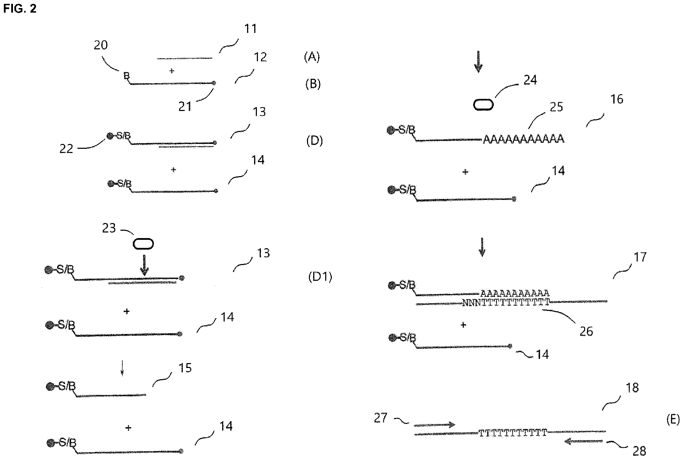

Fig. 2 shows a reaction scheme for an embodiment of the inventive method with cleavage of the hybridization product by an endonuclease. In step (A), a cell lysate containing gapmer 11 is provided, to which RNA-stabilizing agents were added. Gapmer 11 is a synthetic single stranded nucleic acid molecule having a central DNA segment flanked by short RNA segments. In step (B), a specific single-stranded RNA 12 is added to the sample, which is capable of hybridization with gapmer 11 due to a complementary nucleic acid sequence. The RNA 12 is biotinylated ("B") at the 5'-end 20 and comprises a modification 21 at the 3'-end which prevents polyadenylation. The sample is heated to denature annealed nucleic acids, followed by cooling to a temperature, such that hybridization product from gapmer 11 and single-stranded RNA 12 is formed. In step (D), streptavidin beads 22 are added to the sample and conditions are adjusted such that the biotinylated hybridization product binds to the beads 13. Non-hybridized single-stranded RNA, which is also biotinylated, binds simultaneously to the beads 14.

The streptavidin beads with bound hybridization product and single-stranded RNA 13, 14 are separated from the sample, followed by washing. In step (D1), RNase H 23 is added to the nucleic acids bound to the beads 13, 14 and cleaving conditions are adjusted. The RNAse H specifically cleaves RNA/DNA hybridization product 13, whereas single-stranded RNA 14 remains unaffected. The time span of the enzymatic treatment is controlled such that one cleavage per hybridization product occurs on average. As a result, one truncated single-stranded RNA molecule 15 is obtained for each hybridization product 13. In contrast, the residual single-stranded RNA 14 maintains its original structure and nucleic acid sequence, including the 3'-end modification which prevents polyadenylation.

Gapmer 11 is eluted from the solution comprising the beads and nucleic acids bound thereto and discarded, followed by washing. In a subsequent step, beads with bound nucleic acids are subjected to polyadenylation with polyadenylate polymerase and chemicals required for synthesis, which are added in this step. Only truncated single-stranded DNA 15 is polyadenylated to obtain RNA 16 comprising poly(A) tail 25. The original single-stranded RNA 14 is not polyadenylated due to the 3'-end-modification. Subsequently, beads with bound nucleic acids are subjected to cDNA synthesis in the presence of reverse transcriptase and a poly(dT) RT-primer with a universal sequence tag to obtain cDNA26 through intermediate 17. Again, the original single-stranded RNA 14 does not react due to the absence of a poly(A) tail. Cleavage with RNase H had not been specific for a defined RNA/DNA site of the hybridization product. Therefore, the terminal nucleotide of the cleavage site in the truncated RNA molecule 15 cannot be predicted accurately. For this reason, the three terminal nucleotides of the cDNA primer are made by mixed base synthesis including all four nucleotides. These three terminal nucleotides are immediately adjacent to a stretch of poly(dT). At the 5'-end of the poly(dT) stretch of the cDNA primer, there is a universal sequence which defines the reverse PCR primer. Accordingly, it can be ensured that each truncated single stranded RNA is transcribed into a cDNA.

Subsequently, the nucleic acids are eluted from the beads. In step (E), a quantitative PCR is performed with the eluate, such that the cDNA is the PCR template. PCR reagents and enzymes are added together with primers 27, 28, which bind to the cDNA and form PCR intermediate 18. The amount of amplified DNA in the PCR is indicative of the amount of cDNA template, which itself correlates to the amount of original gapmer in the biological sample. The result can be normalized by carrying out in parallel a comparative reaction with a known amount of gapmer. Thereby, potential errors can be eliminated, for example due to potential loss of beads during the process steps.

## Claims

1. A method for determining the level of an antisense oligonucleotide in a biological sample, comprising the steps of
(A) providing a sample, which is the biological sample or a fraction thereof,
(B) adding a first nucleic acid to the sample, which is capable of hybridizing with the antisense oligonucleotide,
(C) forming a hybridization product of the antisense oligonucleotide and the first nucleic acid,
(D) selectively separating the hybridization product from the biological sample,
(E) performing a polymerase chain reaction (PCR) to obtain an amplified nucleic acid, and
(F) determining, based on the level of the amplified nucleic acid, the level of the antisense oligonucleotide in the biological sample,
wherein the hybridization product in step (C) does not comprise two contiguous sequences of nucleotides bound to the antisense oligonucleotide, which are interrupted by a nick.

2. The method of claim 1, wherein the biological sample is selected from tissue, body fluids, such as blood, saliva, urine or liquor, and cells, and/or wherein the antisense oligonucleotide comprises 10 to 30 nucleotides.

3. The method according to at least one of the preceding claims, wherein the first nucleic acid comprises a label for affinity binding, preferably a biotin label, and/or wherein in step (D) the hybridization product is separated from the sample by affinity binding.

4. The method according to at least one of the preceding claims, wherein the amount of the target DNA for the PCR is equivalent to the amount of antisense oligonucleotide in the sample.

5. The method according to at least one of the preceding claims, wherein in step (E) the PCR is quantitative and/ or real-time PCR.

6. A method for determining the level of an antisense oligonucleotide in a biological sample, comprising the steps of
(A) providing a sample, which is the biological sample or a fraction thereof,
(B) adding a duplex DNA to the sample, the duplex DNA comprising a first and second DNA strand, wherein the first strand is capable of hybridizing with the antisense oligonucleotide,
wherein the duplex DNA is added in a molar excess over the antisense oligonucleotide,
(C) forming a hybridization product of the antisense oligonucleotide with the first DNA strand, such that also a displaced second DNA strand is formed,
(D) selectively separating the hybridization product from the sample,
(E) performing a polymerase chain reaction (PCR), wherein the target is the displaced second strand of the duplex DNA, and
(F) determining, based on the result of the PCR, the level of the antisense oligonucleotide in the biological sample.

7. The method of claim 6, wherein the antisense oligonucleotide and/or the first DNA strand comprise one or more, preferably from 2 to 10, more preferably from 4 to 8 locked nucleic acids (LNA).

8. The method of claim 6 and/or 7, wherein in step (B) the first strand of the duplex DNA comprises a first label for affinity binding, preferably a biotin label, and/or wherein in step (D), the hybridization product and duplex DNA are separated from the sample by a first affinity binding step.

9. The method of claim 8, wherein the second nucleic acid comprises a second label for affinity binding, preferably a poly-dA-tail, and/or wherein before step (E), the displaced second strand of the duplex DNA is separated from the sample by a second affinity binding step.

10. A method for determining the level of an antisense oligonucleotide in a biological sample, wherein the antisense oligonucleotide comprises a DNA segment, comprising the steps of
(A) providing a sample, which is the biological sample or a fraction thereof,
(B) adding a single stranded RNA to the sample,
(C) forming a hybridization product from the antisense oligonucleotide and the single stranded RNA,
(D) selectively separating the hybridization product from the sample,
(D1) contacting the hybridization product with an endonuclease, which specifically cleaves the single stranded RNA in the hybridisation product, but not the single stranded RNA which is not part of a hybridization product,
(E) performing a polymerase chain reaction (PCR), and
(F) determining, based on the result of the PCR, the level of the antisense oligonucleotide in the biological sample.

11. The method of claim 10, wherein antisense oligonucleotide is a gapmer and/or wherein the endonuclease is RNase H.

12. The method of claim 11, wherein the single stranded RNA comprises a label for affinity binding, preferably a biotin label, and/or wherein in step (D) the hybridization product is separated from the sample by affinity binding.

13. The method of at least one of claims 10 to 12, wherein the single stranded RNA comprises a modification at the 3'-end which prevents polyadenylation, and/or wherein after step (D1) the cleaved single stranded RNA is polyadenylated at the 3-end.

14. The method of claim 13, wherein cDNA is synthesized from the polyadenylated cleaved single stranded RNA and the cDNA is used as the template in the PCR reaction in step (E).

15. A kit for determining the level of an antisense oligonucleotide in a biological sample in a method of any of the preceding claims, comprising
(i) a first nucleic acid, which is capable of hybridizing with the antisense oligonucleotide and/or means for its synthesis or modification, and
(ii) enzymes, reagents and/or devices for carrying out the method.
